# EUROPEAN PATENT SPECIFICATION

(11) **EP 0 414 179 B1**
(45) Date of publication and mention of the grant of the patent: **08.11.1995**
(21) Application number: 90115884.0
(22) Date of filing: 20.08.1990
(51) Int. Cl.: B01L 3/14, A61B 5/117

(54) **Fluid sample container**
Behälter für Flüssigkeitsproben
Tube d'essai pour des échantillons de liquides

(30) Priority: 21.08.1989 JP 214565/89
(43) Date of publication of application: 27.02.1991
(73) Proprietor: TERUMO KABUSHIKI KAISHA, Tokyo 151 (JP)
(72) Inventor: Kasai, Masaaki, c/o Terumo Kabushiki Kaisha, Nakakoma-gun, Yamanashi-ken (JP)
(74) Representative: Casalonga, Axel

(56) References cited:
- FR-A- 2 339 851
- FR-A- 2 526 169
- US-A- 3 898 046
- US-A- 4 207 990
- US-A- 4 705 551

## Description

### BACKGROUND OF THE INVENTION

### 1. Field of the Invention:

The present invention relates to a fluid sample container, and more particularly to a container for collecting a fluid sample, the container having means for allowing the type of a collected fluid such as blood and fluid test items to be easily identified visuallly from outside the container.

### 2. Description of the Prior Art:

Hospitals and other medical institutions have an established blood sampling system for collecting blood samples to be used in clinical examinations and the like. In such a blood sampling system, necessary blood samples are taken from a patient or blood donor by a single blood sampling needle, known as a multiple sampling needle, mounted on a blood sample tube holder, into different blood sample tubes for the examination of blood serums, blood cells, blood sugar, the agglutinins.

Each of the blood sample tubes, e.g., evacuated blood sample tubes, generally comprise a bottomed tube of glass or plastic whose open end is openably closed by a rubber plug, or a bottom tube of glass or plastic with its open end covered with a sealing member such as a sealing film. Evacuated blood sample tubes which are allotted to different examination types or items are identified by different colors on the rubber plugs or sealing members.

Therefore, the evacuated blood sample tubes can be selected on the basis of the identification according to different color markings on the rubber plugs or sealing members. A selected evacuated blood sample tube is used as follows: First, a multiple sampling needle is mounted on a blood sample tube holder, and its tip end is inserted into a blood vessel of a human body where a blood sample is to be taken. Then, the selected evacuated blood sample tube is introduced into the blood sample tube holder until the evacuated blood sample tube communicates with the blood vessel through the multiple needle. Now, blood flows from the blood vessel through the multiple sampling needle into the evacuated blood sample tube under vacuum.

When the rubber plug or sealing member with the color identification marking is removed at the time the evacuated blood sample tube is in use, the user may occasionally get confused as to the type of the evacuated blood sample. If this happens, the collected blood sample may be examined again or the result of the examination may turn out to be uncertain. A tube with identification markings in its upper portion is disclosed in FR-A-2339851

### SUMMARY OF THE INVENTION

It is a general object of the present invention to provide a blood sample container which allows an identification marking indicating a test item, a container type, or the like to be easily checked visually from outside the container before or after the blood sample container is used.

Another object of the present invention is to provide a blood sample container which has an identification marking indicating a test item, a container type, or the like, the identification marking being prevented from being concealed from view or from being made inconspicuous by a fluid contained in the blood sample container.

Still another object of the present invention is to provide a blood sample container which has an identification marking that can reliably represent many test items or container types, so that blood sample containers with such identification markings cannot be mistaken.

It is also an object of the present invention to provide a fluid sample container comprising a bottomed container having an open end and a closed end, a sealing member sealing the open end of the bottomed container, and identification marking means on the bottomed container near the open end, for identifying a fluid to be collected in the bottomed container or a test item to be conducted on the fluid.

Another object of the present invention is to provide the fluid sample container wherein the identification marking means comprises a first identification marking and a second identification marking, both of the first and second identification markings being positioned near the open end.

Still another object of the present invention is to provide the fluid sample container wherein the first identification marking is positioned in a region which extends from the open end over a length which is 1/3 of the entire length of the bottomed container.

Yet another object of the present invention is to provide the fluid sample container wherein the second identification marking is positioned in a region which extends from the open end over a length which is 1/10 of the entire length of the bottomed container.

Yet still another object of the present invention is to provide the fluid sample container wherein the first identification marking comprises a letter and/or a figure.

A further object of the present invention is to provide the fluid sample container wherein the second identification marking comprises a color code.

A still further object of the present invention is to provide the fluid sample container wherein the letter and/or figure is marked with an ultraviolet-curable ink.

A yet further object of the present invention is to provide the fluid sample container wherein the color code is marked with an ultraviolet-curable ink.

A yet still further object of the present invention is to provide the fluid sample container wherein the bottomed container comprises an evacuated blood sample tube.

The above and other objects, features and advantages of the present invention will become more apparent from the following description when taken in conjunction with the accompanying drawings in which preferred embodiments of the present invention are shown ty way of illustrative example.
FIGS. 1(a) and 1(b) are side elevational views of fluid sample containers according to various embodiments of the present invention;
FIG. 2 is a front elevational view of a blood sample container when it is in use;
FIG. 3 is a cross-sectional view taken along line III - III of FIG. 2;
FIG. 4 is an exploded perspective view showing how a multiple needle and an evacuated blood sample tube, as a blood sample container of the present invention, are inserted into a blood sample tube holder;
FIG. 5 is a perspective view showing the manner in which a blood sample is taken from a human body using a fluid sample container of the present invention; and
FIG. 6 is a perspective view of a plurality of fluid sample containers held in a container stand.

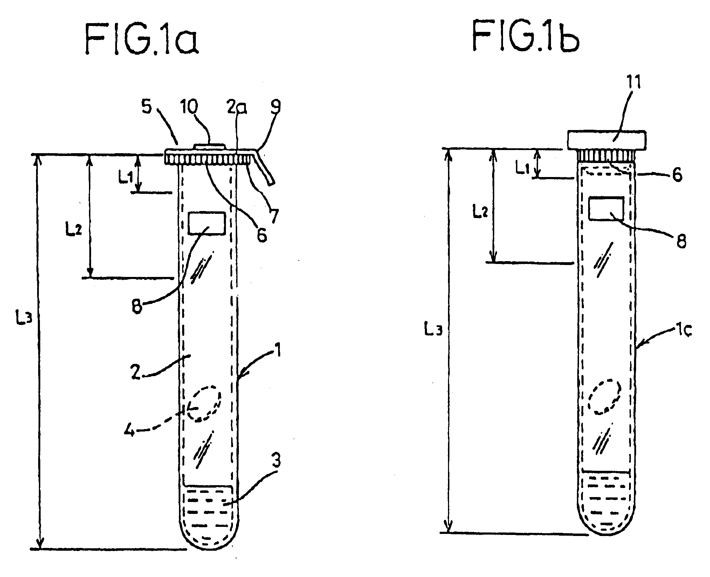

FIG. 1(a) shows a fluid sample container 1 according to an embodiment of the present invention. The fluid sample container 1 comprises a bottomed tube 2 having an open end 2a and a closed end, a blood serum separator 3 disposed in the bottomed tube 2, a piece 4 of nonwoven fabric coated with an agglutination accelerator, which piece 4 is disposed in the bottomed tube 2, and a sealing member 5 covering the open end 2a of the bottomed tube 2 and sealingly closing the interior of the bottomed tube 2.

The interior of the bottomed tube 2 sealed by the sealing member 5 may not necessarily be evacuated, but should preferably evacuated depending on the amount of blood to be sampled. In this embodiment, the bottomed tube 2 is evacuated, and the fluid sample container 1 is used as an evacuated blood sample tube.

As shown in FIG. 1(a), the bottomed tube 2 is in the form of a test tube and is made of a plastic material having a gas barrier capability, such as glass, acrylonitrile, polyethylene terephthalate, or the like. A color code 6 is coated as an identification marking on the region of the outer peripheral surface of the bottomed tube 2 which region extends from the open end 2a over a length L1 which is about 1/10 of the entire length L3 of the bottomed tube 2. In the embodiment illustrated in FIG. 1(a), the color code 6 is coated on the entire peripheral surface of a flange 7 disposed around the open end 2a within the length L1. The color code 6 is of a particular color depending on the kind of a test to be carried out on a flood or blood sample collected in the evacuated blood sample tube 1, so that the test item for the blood sample tube 1 can be identified according to the color of the color code 6. The color code 6 is colored with an ultraviolet-curable ink (such like a product New dye cure ZTER from Dai-Nippon Ink Co. Ltd.) which can be set due to cross-linking upon exposure to ultraviolet radiation.

Another identification marking 8, which may comprise letters, a figure, or the like, is applied to the region of the outer peripheral surface of the bottomed tube 2 which region extends from the open end 2a over a length L2 which is about 1/3 of the entire length L3 of the bottomed tube 2. Generally, the identification marking 8 is used as a marking indicative of any information which cannot easily be represented by the color code 6. The identification marking 8 is also marked with an ultraviolet-curable ink.

The blood serum separator 3 serves to block blood clots centrifugally after the sampled blood is stored in the evacuated blood sample tube 1. The agglutination of the collected blood sample is accelerated by the agglutination accelerator coated on the nonwoven fabric piece 4.

The sealing member 5 comprises a film 9 having a gas barrier capability The film 9 may be made of an aluminum foil, a film with an aluminum layer deposited thereon by evaporation, or a film coated with a gas barrier material such as polyvinylidene chloride, polyvinyl alcohol, ethylene-vinyl alcohol, or the like. To apply the sealing member 5 to the evacuated blood sample tube 1, an adhesive layer may first be applied to the evacuated blood sample tube 1, and then the sealing member 5 may be bonded to the adhesive layer.

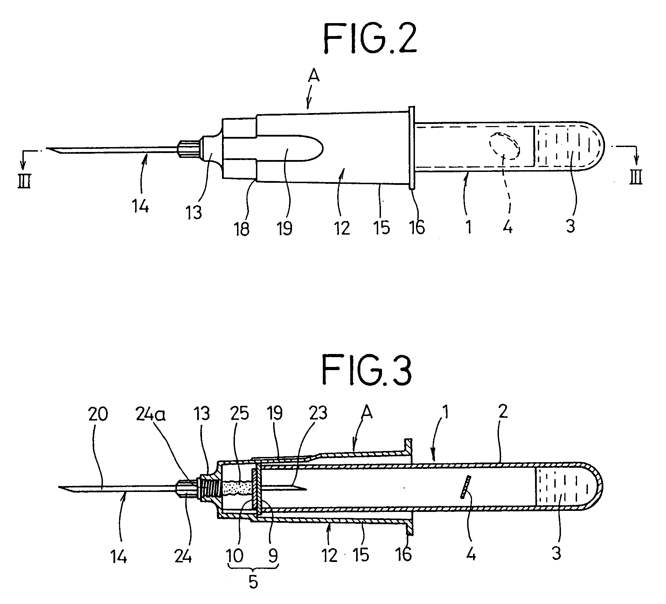

To the sealing member 5, there is applied a resealable rubber member 10. The resealable rubber member 10 may be made of synthetic rubber, natural rubber, or thermoplastic elastomer. After a multiple sampling needle 14 (see FIG. 2) has pierced the resealable rubber member (10) and is pulled away, the resealable rubber member 10 sealingly close the interior of the evacuated blood sample tube 1 again due to its own resiliency.

FIG. 1(b) shows an evacuated fluid sample container or blood sample tube 1c according to an other embodiment of the present invention.

The evacuated blood sample tube 1c shown in FIG. 1(b) includes a rubber plug 11 fitted in the open end of the bottomed tube 2, and has both the color code 6 and the identification marking 8 as with the evacuated blood sample tube 1 shown in FIG. 1(a). According to an alternative of the blood sample tube 1, a rubber plug 11 may be fitted in the bottomed tube 2, and the color code 6 or the identification marking 8 may singly be coated or applied.

Each of the evacuated blood sample tubes 1 and 1c is used in a blood sampling instrument A as shown in FIGS. 2 and 3. The blood sampling instrument A comprises a blood sample tube holder 12, a multiple sampling needle (blood sampling needle) 14 attached to a needle joint 13 on one end of the blood sample tube holder 12, and the evacuated blood sample tube 1 which is inserted into the blood sample holder 12 through the other open end thereof.

The blood sample tube holder 12 is made of a plastic material such as polypropylene. The blood sample tube holder 12 has a substantially cylindrical holder body 15 having an open end through which the evacuated blood sample tube 1 is inserted. The holder body 15 has a flange 16 around the open end thereof which can be held by the user to facilitate a blood sampling process. The needle joint 13 is integrally formed with the other end of the holder body 15. The holder body 15 has a diameter which is progressively smaller from the flange 16 toward the needle joint 13, with a step 18 provided near the needle joint 13, defining a reduced-diameter portion contiguous to the needle joint 13. The holder body 15 also has three radially inwardly projecting wall portions 19 on its inner wall surface which extend from a substantially longitudinally central portion toward the needle joint 13, the wall portions 19 being 120° spaced apart. When the evacuated blood sample tube 1 is inserted into the holder body 15, it is engaged by the bulging wall portions 19 with gradually increasing forces, so that the evacuated blood sample tube 1 can smoothly be fitted into the holder body 15. The bulging wall portions 19 are thinner than the rest of the holder body 15 so that when the evacuated blood sample tube 1 is inserted into the holder body 15, the top surfaces or radially innermost surfaces of the bulging wall portions 19 are engaged and slightly elastically deformed by the evacuated blood sample tube 1, thereby applying reactive forces to the decompressed blood sample tube 1. Therefore, when the evacuated blood sample tube is inserted into the blood sample tube holder 12, the bulging portions 19 are elastically deformed, applying gradually increasing resilient fitting forces to the evacuated blood sample tube 1. After the evacuated blood sample tube 1 is inserted over a certain length, it is securely fitted in the blood sample tube holder 12 under the resilient reactive forces from the bulging wall portions 19.

As shown in FIGS. 2 and 3, the evacuated blood sample tube 1 is used when it is inserted into the blood sample tube holder 12 with the multiple sampling needle 14 attached thereto. The multiple needle 14 comprises a cannula 20 for piercing a blood vessel, a rubber piercing member 23 for piercing the resealable rubber member 10 and the sealing film 9, and a needle base 24 which supports the cannula 20 and the rubber piercing member 23. The needle base 24 is externally threaded at 24a near the rubber piercing member 23 so that the needle base 24 can be threaded into the blood sample tube holder 12. As shown in FIGS. 2 and 3, the multiple needle 14 is threaded into the needle joint 13 of the blood sample tube holder 12. The rubber piercing member 23 is covered with a thin rubber tube referred to as a rubber tip.

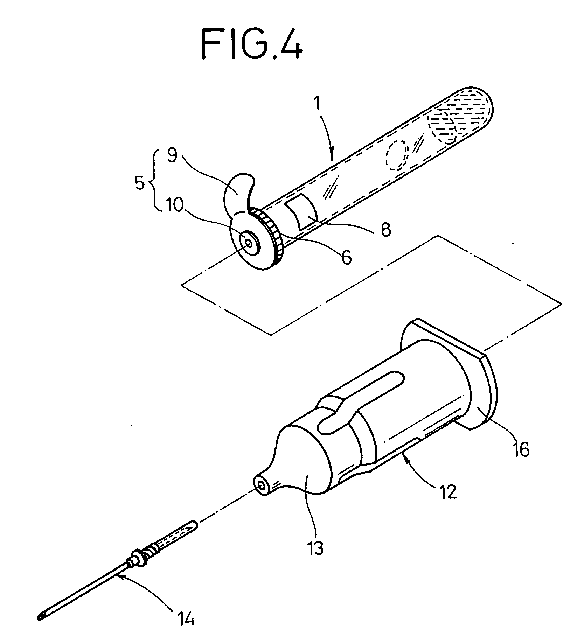

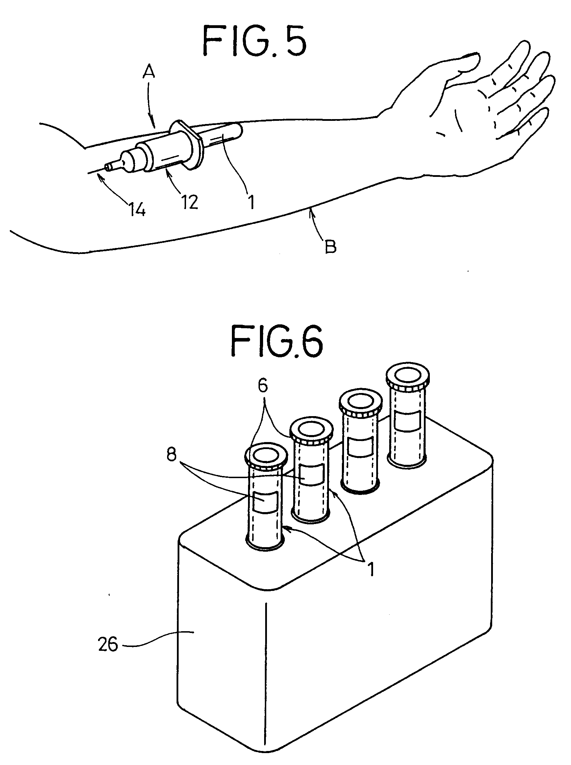

The evacuated blood sample tube 1 (1c) is used as follows:
The evacuated blood sample tube 1 allotted to a test item which is required to carried out is selected according to the color code 6 and/or the identification marking 8. Then, the multiple sampling needle 14 is threaded into the blood sample tube holder 12 (see FIG. 4). Thereafter, the cannula 20 of the multiple sampling needle 14 is inserted into a blood vessel of a human body part B (see FIG. 5) such as an arm of the blood donor. Then, the evacuated blood sample tube 1 is introduced into the holder body 15 of the blood sample tube holder 12 until the evacuated blood sample tube 1 abuts against the bulging wall portions 19 of the holder body 15. When the evacuated blood sample tube 1 is further inserted, it is forced into the holder body 15 against resilient reactive forces from the bulging wall portions 19. The evacuated blood sample tube 1 as it goes into the holder body 15 is therefore subjected to progressively increasing forces while it is being inserted. Then, the rubber piercing member 23 pierces the resealable rubber member 10 and the sealing film 9. Since the blood vessel and the evacuated blood sample tube 1 are now brought into fluid communication with each other, blood is drawn from the blood vessel into the evacuated blood sample tube 1 at a rate dependent on the vacuum developed therein. After the blood has been sampled in the evacuated blood sample tube 1, a label indicating the name of the blood donor is attached to the evacuated blood sample tube 1, and the evacuated blood sample tube 1 is temporarily supported in a tube stand 26 (FIG. 6) with the sealing member 5 being peeled off. The evacuated blood sample tube 1 in the tube stand 26 can easily be identified for the type of the examination to be conducted on the collected blood sample, according to the color code 6 and/or the identification marking 8.

While the fluid sample container or blood sample tube is in the form of a test tube in the illustrated embodiment, it is not limited to the test tube configuration, but may be of any of various configurations such as a bottle.

With the present invention, as described above, since the identification marking is applied to the region of the outer peripheral surface of the bottom tube which region extends from the open end thereof over about 1/3 of the entire length of the bottomed tube, the identification marking will not be accidentally peeled off before or after the fluid sample container is used, and is always kept in a position above the level of the sampled fluid or blood in the fluid sample container. Therefore, even after the rubber plug or sealing member of film has been peeled off, the user can identify the type of the examination to be conducted on the blood contained in the fluid sample container according to the identification marking. The identification marking positioned above the collected blood will not be rendered inconspicuous by the color of the blood.

If the color code is employed as an identification marking, because it is applied to the region of the outer peripheral surface of the bottomed tube which region extends from the open end thereof over a length that is about 1/10 of the entire length of the bottomed tube, the fluid sample container can easily be identified even when it is held in a container stand or the like.

## Claims

1. A fluid sample container (1) comprising:
a bottomed container (2) of circular cross-section having an open end (2a) and a closed end;
a sealing member (5) sealing the open end of said bottomed container; and
identification marking means (6,8) directly readable by a user and positioned on the outer surface of said bottomed container near said open end, for identifying a fluid to be collected in said bottomed container or a test item to be conducted on the fluid to be collected, characterized in that said identification marking means comprises a first identification marking (6) and a second identification marking (8), made of UV-curable ink, said second identification marking (8) being positioned beneath said first identification marking (6) and in a region which extends from said open end over a length which is about 1/3 of the entire length of said bottomed container, said first identification marking (6) being positioned in a region which extends from said open end over a length which is about 1/10 of the entire length of said bottomed container, said first identification marking (6) comprising a color code, and said second identification marking (8) comprising an indicia.

2. A fluid sample container according to claim 1, wherein said second identification marking (8) comprises a letter and/or figure.

3. A fluid sample container according to anyone of the preceding claims, wherein said bottomed container (2) comprises an evacuated blood sample tube.

## Patentansprüche

1. Flüssigkeitsprobenbehälter (1), umfassend:
- einen mit Boden versehenen Behälter (2) mit kreisförmigem Querschnitt, der ein offenes Ende (2a) und ein geschlossenes Ende aufweist;
- ein Verschlußelement (5), das das offene Ende des mit Boden versehenen Behälters abdichtet; und
- eine Identifizierungsmarkierungseinrichtung (6, 8), die durch einen Benutzer direkt lesbar ist und an der Außenfläche des mit Boden versehenen Behälters nahe dem offenen Ende angeordnet ist, um eine in dem mit Boden versehenen Behälter oder einen auf der zu sammelnden Flüssigkeit zu führenden Testgegenstand zu identifizieren, dadurch **gekennzeichnet,** daß die Identifizierungsmarkierungseinrichtung eine erste Identifizierungsmarkierung (6) und eine zweite Identifizierungsmarkierung (8), hergestellt aus UV-behandelbarer Farbe, umfaßt, wobei die zweite Identifizierungsmarkierung (8) unterhalb der ersten Identifizierungsmarkierung (6) und in einem Bereich angeordnet ist, der sich von dem offenen Ende über eine Länge erstreckt, die etwa 1/3 der Gesamtlänge des mit Boden versehenen Behälters ist, wobei die erste Identifizierungsmarkierung (6) in einem Bereich angeordnet ist, der sich von dem offenen Ende über eine Länge erstreckt, die etwa 1/10 der Gesamtlänge des mit Boden versehenen Behälters ist, wobei die erste Identifizierungsmarkierung (6) einen Farbcode umfaßt und wobei die zweite Identifizierungsmarkierung (8) eine Anzeige umfaßt.

2. Flüssigkeitsprobenbehälter nach Anspruch 1, bei dem die zweite Identifizierungsmarkierung (8) einen Buchstaben und/oder Zeichnung umfaßt.

3. Flüssigkeitsprobenbehälter nach einem beliebigen der vorhergehenden Ansprüche, bei der der mit Boden versehene Behälter (2) ein evakuiertes Blutprobenrohr umfaßt.

## Revendications

1. Récipient (1) pour échantillon de liquide, qui comprend :
- un récipient (2) muni d'un fond, de section circulaire et comportant une extrémité ouverte (2a) et une extrémité fermée,
- un élément d'étanchéité (5) qui scelle l'extrémité ouverte du récipient muni d'un fond, et
- des moyens (6, 8) formant repères d'identification qui peuvent être lus directement par l'utilisateur et qui sont placés sur la surface extérieure dudit récipient muni d'un fond, près de ladite extrémité ouverte, lesdits moyens servant à identifier un liquide à recueillir dans ledit récipient ou un type d'essai à effectuer sur le fluide à recueillir,
caractérisé en ce que lesdits moyens formant repères d'identification comprennent un premier repère d'identification (6) et un second repère d'identification (8) faits d'une encre durcissable par rayonnement ultraviolet, ledit second repère d'identification (8) étant placé endessous dudit premier repère d'identification (6) et dans une région qui s'étend depuis ladite extrémité ouverte sur une longueur qui vaut environ un tiers de la longueur totale dudit récipient muni d'un fond, ledit premier repère d'identification (6) étant placé dans une région qui s'étend depuis ladite extrémité ouverte sur une longueur qui vaut environ un dixième de la longueur totale dudit récipient muni d'un fond, ledit premier repère d'identification (6) comprenant un code coloré et ledit second repère d'identification (8) comprenant un indice.

2. Récipient pour échantillon de liquide selon la revendication 1, dans lequel ledit second repère d'identification (8) comprend une lettre et/ou un nombre.

3. Récipient pour échantillon de liquide selon l'une quelconque des précédentes revendications, dans lequel ledit récipient (2) muni d'un fond est un tube pour échantillon de sang dans lequel on a fait le vide.
